# FASCICULE DE BREVET EUROPEEN

(11) **EP 0 687 459 B1**
(45) Date de publication et mention de la délivrance du brevet: **28.08.1996**
(21) Numéro de dépôt: 95401085.6
(22) Date de dépôt: 10.05.1995
(51) Int. Cl.: A61K 7/42, A61K 7/06, C09D 133/12, C08L 33/12

(54) **Composition capillaire comprenant une dispersion aqueuse de polymères**
Haarpflegemittel, das eine wässrige Polymer-Dispersion enthält
Hair composition comprising an aqueous polymer dispersion

(30) Priorité: 17.06.1994 FR 9407479
(43) Date de publication de la demande: 20.12.1995
(73) Titulaire: L'OREAL, F-75008 Paris (FR)
(72) Inventeur: Lion, Bertrand, F-93190 Livry Gargan (FR); Mondet, Jean, F-93700 Drancy (FR)
(74) Mandataire: Dodin, Catherine

(56) Documents cités:
- EP-A- 0 478 284
- US-A- 4 300 580
- RESEARCH DISCLOSURE, vol.326, 1991, NEW YORK (USA) pages 390 - 391 DISCLOSED ANONYMOUSLY 'Hair Grooming Compositions'

## Description

La présente invention a trait à une composition capillaire comprenant en tant qu'agent filmogène, une dispersion aqueuse de polymères, ainsi qu'à l'utilisation d'une telle dispersion dans une composition capillaire.

Il est connu d'utiliser des dispersions aqueuses de polymère en tant qu'agent filmogène, dans des compositions capillaires.
Les propriétés des dispersions aqueuses ainsi obtenues dépendent de la nature des polymères, et donc des monomères, à partir desquels elles sont préparées. Il peut toutefois être intéressant de pouvoir modifier légèrement ces propriétés, par exemple en accentuant/optimisant une propriété particulièrement intéressante, ou en en développant une nouvelle que ladite dispersion ne pourrait avoir de par sa composition propre.

L'invention a pour but de proposer une composition capillaire comprenant une dispersion aqueuse d'un polymère choisi dans le groupe constitué par les polyesters, les polyesteramides et les alkydes, présentant des propriétés améliorées par rapport aux dispersions de polyesters, polyesteramides et/ou alkydes de l'art antérieur.

Un objet de l'invention est donc une composition capillaire comprenant une dispersion aqueuse de polymère constituée par des particules résultant de la polymérisation radicalaire d'au moins un monomère radicalaire à l'intérieur et/ou partiellement en surface, de particules préexistantes d'au moins un polymère choisi dans le groupe constitué par les polyesters, les polyesteramides et les alkydes. Un autre objet de l'invention est l'utilisation d'une dispersion aqueuse de polymère constituée par des particules résultant de la polymérisation radicalaire d'au moins un monomère radicalaire à l'intérieur et/ou partiellement en surface, de particules préexistantes d'au moins un polymère choisi dans le groupe constitué par les polyesters, les polyesteramides et les alkydes, comme agent filmogène dans une composition capillaire.

Dans la suite de la présente description, on entend par "polyester", tout polymère, seul ou en mélange, choisi dans le groupe constitué par les polyesters, les polyesteramides et les alkydes.

On a constaté que l'utilisation dans des compositions capillaires de dispersions aqueuses selon l'invention, c'est-à-dire de dispersions aqueuses de polymères hybrides de polyesters, permet d'obtenir une composition présentant des propriétés particulières, propriétés qu'il n'est pas possible d'obtenir en utilisant, par exemple, un simple mélange de dispersions aqueuses préexistantes de polyester et de polymère acrylique et/ou vinylique.

Le document Research Disclosure 32613, vol.326, 1991, New York, p. 390-391, decrit une composition capillaire comprenant un mélange d'un polymère soluble et d'un polymère de type polyester ou polyesteramide dispersible dans l'eau.

Un avantage de la présente invention est donc de pouvoir, à partir d'une dispersion aqueuse de polymère déjà existante, développer et/ou optimiser certaines propriétés particulièrement intéressantes, de manière relativement contrôlée.

Afin de préparer la composition capillaire selon l'invention, on prépare tout d'abord une dispersion aqueuse de polyester.
Cette dispersion peut être préparée par l'homme du métier sur base de ses connaissances techniques générales, en particulier de la manière suivante.
Lorsque le polymère de polyester est insoluble dans l'eau, on peut le dissoudre dans un solvant organique faiblement soluble dans l'eau, ajouter de l'eau de manière à former une émulsion, puis évaporer le solvant organique de manière à obtenir une dispersion aqueuse du polymère de polyester dans l'eau présentant un taux de matière sèche d'environ 30-50% en poids.
Lorsque le polymère de polyester est autodispersible dans l'eau, cette étape peut être évitée si le polymère comporte suffisamment de groupes hydrophiles.

La dispersion aqueuse de polyester utilisée peut être une dispersion aqueuse de polyester anionique, cationique, non ionique ou amphotère, de polyesteramides, d'alkydes c'est-à-dire de polyesters à chaîne grasse, seul ou en mélange.
La dispersion peut également être une dispersion de polyesters à groupement ionisables latéraux, tels que sulfonique ou carboxylique.
Le polyester peut comporter des groupements insaturés, par exemple lorsqu'il est obtenu par polycondensation d'un diol ou d'une diamine avec un anhydride d'acide insaturé, l'anhydride maléique par exemple. La charge du monomère radicalaire peut dans ce cas réagir ultérieurement avec le polyester insaturé et donner lieu à un greffage et/ou à une réticulation. On obtient ainsi une dispersion d'un polymère hybride greffé et/ou réticulé, qui peut procurer au film obtenu après application de la dispersion, des propriétés mécaniques particulières, telles qu'une amélioration de l'adhérence dudit film.

La dispersion aqueuse de polymères hybrides de polyester selon l'invention est obtenue par polymérisation radicalaire d'au moins un monomère à l'intérieur et/ou partiellement en surface de particules préexistantes de polyester.

Le monomère radicalaire peut être de nature vinylique ou acrylique, et peut être anionique, cationique, non ionique ou amphotère. On peut également utiliser un mélange de monomères de nature différente.
Le monomère, ou le mélange de monomères, est, de préférence, insoluble ou faiblement soluble dans l'eau.
Parmi les monomères susceptibles d'être employés, on peut citer les esters d'acide acrylique ou méthacrylique, tels que les acrylate ou méthacrylate de méthyle, d'éthyle, de propyle, de butyle, d'isobutyle, de tertiobutyle, et d'éthyl-2-hexyle; les acrylamides ou méthacrylamides N-substituées ou N,N-substituées; les esters vinyliques tels que l'acétate de vinyle; le styrène.
On peut également utiliser, seul ou en mélange, un monomère vinylique, acrylique ou méthacrylique comportant un ou plusieurs groupes siloxanes, en particulier
. le monomère de formule CH₂=C(CH₃)-C(O)-O-(CH₂)₃-Si-[O-Si(CH₃)₃]₃
. un macromonomère siliconé à terminaison monofonctionnelle vinylique, allylique, ester éther ou amide de l'acide acrylique ou méthacrylique, de formule

   CH₂=C(R1)-C(O)-X-(CH₂)ₚ-[Si(CH₃)(R4)-O-]ₙ-Si(CH₃)₂-R3

   dans laquelle R1 représente H ou CH₃, X représente O ou NH, p est un entier pouvant être nul, R3 et R4 représentent de manière indépendante CH₃ ou un groupe aliphatique, cycloaliphatique ou aromatique, et n est un entier, de préférence compris entre 3 et 300.
On peut également utiliser un monomère vinylique, allylique, ester éther ou amide de l'acide acrylique ou métacrylique comportant un ou plusieurs groupes halogénés, en particulier chlorés et/ou fluorés, et/ou comportant un groupe absorbant dans l'UVA et/ou l'UVB et pouvant apporter après polymérisation, une certaine photoprotection contre le rayonnement ultraviolet, en particulier solaire, par exemple les groupements benzylidène camphre et benzotriazole, substitués ou non, parmi lesquels on peut citer le 2-(2'-hydroxy-5-methacrylyloxyéthylphényl)-2-H-benzotriazole.
Lorsque le monomère, ou le mélange de monomères, est sous forme liquide à température ambiante, la polymérisation radicalaire peut être effectuée sans employer de solvant.
Lorsque le monomère, ou le mélange de monomères, est sous forme solide à température ambiante, on peut le dissoudre avant la polymérisation, de préférence dans un solvant organique, par exemple polaire et miscible à l'eau, tel que le méthanol. Dans ce cas, après polymérisation, on peut distiller le solvant organique contenu dans la dispersion aqueuse, si cela est nécessaire.

La préparation des dispersions aqueuse selon l'invention est faite dans des conditions telles que le monomère polymérise à l'intérieur et/ou partiellement en surface des particules du polymère en l'absence de toute nucléation, c'est-à-dire sans qu'il n'y ait de formation de particules nouvelles.
Pour ce faire, on peut introduire le polymère de polyester en dispersion aqueuse présentant un taux de matière sèche de 30-50% en poids dans un réacteur de polymérisation.
On peut alors y ajouter le monomère, ou le mélange de monomère, tel quel ou en solution dans un solvant adéquat, ainsi qu'un amorceur de polymérisation radicalaire.
Selon sa nature, on introduit l'amorceur radicalaire soit sous forme de solution dans un solvant organique, soit sous forme de solution aqueuse, soit encore préalablement dissout dans le mélange de monomères.

Dans le premier cas, il peut être ajouté en même temps que le monomère en solution, et dans le second cas, il peut être ajouté après le monomère.
On peut utiliser un amorceur de polymérisation radicalaire organique, non soluble dans l'eau, de type peroxyde ou percarbonate, tel que le tertiobutylperoxy-2-éthylhexanoate, ou un amorceur organique soluble dans l'eau, ou encore un amorceur minéral tel que le persulfate de potassium.
On prépare donc un mélange aqueux comprenant le polymère de polyester, le monomère et l'amorceur de polymérisation.
On peut également ajouter à ce mélange un stabilisant qui peut être notamment un tensioactif, ou un mélange de tensioactifs, anionique, amphotère, cationique et/ou non ionique. Lorsque le polyester utilisé est lui-même ionique, il est préférable d'utiliser un tensioactif de même caractère ionique ou amphotère.
De préférence, on utilise un tensioactif ionique et polyoxyéthyléné, en une quantité de 0,5-10% en poids de matière sèche par rapport au poids de matière sèche de polyester .
On chauffe alors le mélange jusqu'à la température nécessaire de manière à permettre la décomposition de l'amorceur, et l'on poursuit la polymérisation jusqu'à épuisement des monomères.

Les proportions en monomère radicalaire vis-à-vis du polymère de polyester peuvent être de 10-95% en poids de matière sèche de monomère radicalaire et 5-90% en poids de matière sèche de polyester.

On obtient ainsi une dispersion aqueuse de polymère hybride, dont les particules la constituant se présentent sous forme de particules composites, semblables à un "alliage" des deux polymères de base et de taille comparable à celles des particules de polyester avant polymérisation radicalaire.
Les dispersions ainsi obtenues possèdent des propriétés qui leur sont propres, différentes de celles que l'on obtiendrait en mélangeant deux dispersions aqueuses de chacun des constituants.

Les dispersions selon l'invention peuvent être utilisées comme agent filmogène dans des compositions capillaires telles que des laques ou des shampooings, des lotions de mise en plis, des lotions ou des mousses de coiffage, de la même manière qu'une dispersion aqueuse de polymère selon l'état de la technique.
Ces compositions comprennent les ingrédients habituellement utilisés dans le domaine capillaire, et peuvent être préparées selon les méthodes usuelles connues de l'homme du métier.
On peut également utiliser les dispersions selon l'invention dans des produits destinés à la photoprotection des cheveux contre le rayonnement ultraviolet, en particulier contre le rayonnement solaire, lorsqu'elles contiennent un monomère adéquat, susceptible d'apporter une certaine protection solaire.

L'invention est illustrée plus en détail dans les exemples suivants, dans lesquels les pourcentages sont donnés en poids.

### Exemple 1

On disperse 50 g de granulés solides de polymère de polyester à groupement sulfonique AQ 38 vendu par Eastman Kodak dans 250 ml d'eau permutée préalablement chauffée à 80°C, tout en maintenant une agitation cisaillante à l'aide d'un disperseur de type Moritz.
On obtient une dispersion ayant une taille moyenne des particules de 40 nm avec une polydispersité de 0,15.
On laisse reposer la dispersion pendant 24 heures puis on l'introduit dans un réacteur préalablement chauffé à 80°C; on ajoute 50 g de méthacrylate de méthyle au goutte à goutte, ce qui prend environ 45 minutes, puis on laisse sous agitation pendant 1 heure à 80°C. On ajoute 0,5 ml de tertbutylperoxy-2-éthylhexanoate (Trigonox 21S de Akzo) et on laisse réagir 6 heures sous agitation et barbotage d'azote à 80°C.
Le mélange alors obtenu à la même apparence qu'au départ bien que tout le monomère ait polymérisé. On descend la température du mélange réactionnel à 25°C, on filtre sur toile de Nylon et l'on concentre la dispersion sous pression réduite jusqu'à obtention d'un taux de matière sèche de 40%.
On obtient ainsi une dispersion qui, après une nouvelle filtration, a les caractéristiques suivantes:
. taille moyenne des particules déterminée par un appareil de diffusion quasi-élastique de lumière du type Coulter N4, de Coultronix: 44 nm
. polydispersité: 0,15
   Considérant que la taille des particules dans la dispersion initiale de polyester AQ 38 est de 40 nm (polydispersité : 0,15), on peut donc constater que la polymérisation du monomère n'a presque pas modifié la taille desdites particules initiales.
. absence de double distribution de particules, ce qui signifie que, lors de la polymérisation, on n'a pas créé une seconde population de particules, en plus de la population initiale.

La dispersion obtenue est une dispersion aqueuse d'un polymère hybride dont les particules résultent de la polymérisation radicalaire d'un monomère méthacrylate de méthyle sur et/ou dans les particules d'un polymère préexistant de type polyester à groupement sulfonique.

### Exemples 2 à 5

De manière similaire à celle décrite dans l'exemple 1, on prépare à partir d'une dispersion aqueuse de granulés de polyester sulfonique (AQ 38 de Eastman Kodak), différents polymères hybrides selon le tableau ci-dessous.
L'amorçage est toujours effectué avec 0,5 ml de Trigonox 21S.
Les mesures de taille de particules et de polydispersité sont effectuées pour une dispersion ayant un taux de matière sèche de 40%.

| | polyester | eau ajoutée | monomère | taille des particules | polydispersité |
|---|---|---|---|---|---|
| exemple 2 | 70 g | 250 ml | 30 g de méthacrylate de méthyle | 35 nm | 0,10 |
| exemple 3 | 50 g | 250 ml | 45 g de méthacrylate de méthyle + 5 g de diméthacrylate d'éthylèneglycol | 45 nm | < 0,10 |
| exemple 4 | 70 g | 250 ml | 30 g de méthacrylate d'isobutyle | 45 nm | 0,12 |
| exemple 5 | 70 g | 300 ml | 25 g de méthacrylate de méthyle + 5 g de diméthacrylate d'éthylèneglycol | 32 nm | 0,18 |

On constate, pour tous ces exemples, que l'on obtient une population de particules unique et homogène, dont la taille a été peu modifiée par la polymérisation.

### Exemple 6

On compare les propriétés de filmification des dispersions des polymères selon l'invention, à température ambiante.
On observe que les dispersions des exemples 1, 2, 3 et 5 filmifient lorsqu'on leur ajoute un plastifiant (20 g de monométhyléther de tripropylèneglycol pour 100 g de matière sèche de dispersion) et permettent l'obtention de films homogènes et transparents après séchage.

### Exemple 7

On prépare un spray de fixation, conditionné en flacon-pompe, en conditionnant dans un récipient approprié:

| | |
|---|---|
| . dispersion aqueuse de l'exemple 1 (40% de matière sèche) | 22,5 g |
| . monométhyléther de tripropylèneglycol | 1,8 g |
| . parfum, colorant, conservateur | q.s. |
| . eau | q.s.p. 100 g |

Après préparation du mélange, on dispose sur le récipient une pompe de pulvérisation.

On obtient ainsi un spray de fixation qui permet l'obtention, après pulvérisation sur les cheveux, d'un film aux propriétés adéquates.

### Exemple 8

On prépare une lotion de mise en plis ayant la composition suivante:

| | |
|---|---|
| . dispersion aqueuse de l'exemple 1 (40% de matière sèche) | 25 g |
| . parfum, colorant, conservateur | q.s. |
| . eau | q.s.p. 100 g |

On obtient ainsi une lotion de mise en plis qui permet l'obtention, après application sur les cheveux, d'un film aux propriétés adéquates.

### Exemple 9

De manière similaire à l'exemple 8, on prépare une lotion de mise en plis comprenant une dispersion aqueuse telle que préparée aux exemples 2 à 5, diluée à 5% en poids de taux de matière sèche.
Dans les quatre cas, on obtient une lotion permettant une bonne fixation de la coiffure.

### Exemple 10

On disperse 2,5 g de granulés solides de polymère de polyester à groupement sulfonique AQ 38 vendu par Eastman Kodak dans 200 ml d'eau permutée préalablement chauffée à 80°C, tout en maintenant une agitation cisaillante à l'aide d'un disperseur de type Moritz.
On ajoute 1 g de lauryle sulfate de sodium et 0,25 g de persulfate de potassium. On laisse agiter puis l'on chauffe à 72°C et l'on introduit 47,5 g d'acrylate de tertiobutyle. On maintient 8 heures à 72°C sous agitation. On descend la température du mélange réactionnel à 25°C, on filtre sur toile de Nylon et l'on concentre la dispersion sous pression réduite jusqu'à obtention d'un taux de matière sèche de 20%.
On obtient ainsi une dispersion qui a les caractéristiques suivantes:
. taille moyenne des particules déterminée par un appareil de diffusion quasi-élastique de lumière du type Coulter N4, de Coultronix: 57 nm
. polydispèrsité: 0,15

### Exemple 11

On prépare une lotion de mise en plis ayant la composition suivante:

| | |
|---|---|
| . dispersion aqueuse de l'exemple 10 (5% de matière sèche) | 25 g |
| . parfum, colorant, conservateur | q.s. |
| . eau | q.s.p. 100 g |

On obtient ainsi une lotion de mise en plis permettant une bonne fixation de la coiffure.

### Exemple 12

On prépare une disperse aqueuse de 2,5 g de polymère de polyester à groupement sulfonique AQ 38 d'Eastman Kodak dans 200 ml d'eau permutée. On l'introduit dans un réacteur préalablement chauffé à 80°C; on ajoute 2,5 g de lauryl sulfate de sodium, puis 0,25 g de persulfate de potassium et 0,25 g d'hydrogénocarbonate de sodium.
On chauffe à 72°C puis on ajoute un mélange de 42,75 g d'acrylate de tertiobutyle et 4,75 g de macromonomère siliconé vendu par 3M (poids moléculaire 9000 à 12 000).
On maintient le tout à 72°C sous agitation pendant 24 heures puis on descend la température du mélange réactionnel à 25°C, on filtre sur toile de Nylon et l'on concentre la dispersion sous pression réduite jusqu'à obtention d'un taux de matière sèche de 20%.
La dispersion obtenue est une dispersion aqueuse d'un polymère hybride dont les particules résultent de la polymérisation radicalaire d'un mélange de macromonomère siliconé + acrylate de tertiobutyle, sur et/ou dans les particules d'un polymère préexistant de type polyester à groupement sulfonique.

### Exemple 13

On prépare une lotion de mise en plis ayant la composition suivante:

| | |
|---|---|
| . dispersion aqueuse de l'exemple 12 (5% de matière sèche) | 25 g |
| . parfum, colorant, conservateur | q.s. |
| . eau | q.s.p. 100 g |

On obtient ainsi une lotion de mise en plis permettant une bonne fixation de la coiffure.

### Exemple 14

On prépare une disperse aqueuse de 2,5 g de polymère de polyester à groupement sulfonique AQ 38 d'Eastman Kodak dans 200 ml d'eau permutée. On l'introduit dans un réacteur préalablement chauffé à 80°C; on ajoute 2,5 g de lauryl sulfate de sodium, puis 0,25 g de persulfate de potassium et 0,25 g d'hydrogénocarbonate de sodium.
On chauffe à 72°C puis on ajoute un mélange de 42,75 g d'acrylate de tertiobutyle et 4,75 g d'acrylate de perfluorohexyle (ATOCHEM).
On maintient le tout à 72°C sous agitation pendant 24 heures puis on descend la température du mélange réactionnel à 25°C, on filtre sur toile de Nylon et l'on concentre la dispersion sous pression réduite jusqu'à obtention d'un taux de matière sèche de 20%.
La dispersion obtenue est une dispersion aqueuse d'un polymère hybride dont les particules résultent de la polymérisation radicalaire d'un mélange d'acrylates de perfluorohexyle et de tertiobutyle, sur et/ou dans les particules d'un polymère préexistant de type polyester à groupement sulfonique.

### Exemple 15

On prépare une lotion de mise en plis ayant la composition suivante:

| | |
|---|---|
| . dispersion aqueuse de l'exemple 14 (5% de matière sèche) | 25 g |
| . parfum, colorant, conservateur | q.s. |
| . eau | q.s.p. 100 g |

On obtient ainsi une lotion de mise en plis permettant une bonne fixation de la coiffure.

## Revendications

1. Composition capillaire comprenant une dispersion aqueuse de polymère, constituée par des particules résultant de la polymérisation radicalaire d'au moins un monomère radicalaire à l'intérieur et/ou partiellement en surface, de particules préexistantes d'au moins un polymère choisi dans le groupe constitué par les polyesters, les polyesteramides et les alkydes.

2. Composition selon la revendication 1, dans laquelle le monomère est choisi dans le groupe constitué par les vinyliques et les acryliques.

3. Composition selon l'une des revendications précédentes, dans laquelle le monomère est choisi parmi les esters d'acide acrylique ou méthacrylique; les acrylamides ou méthacrylamides N-substituées ou N,N-substituées; les esters vinyliques; le styrène; les monomères vinyliques, acryliques ou méthacryliques comportant un ou plusieurs groupes siloxanes; les monomères vinyliques, allyliques, ester éther ou amide de l'acide acrylique ou métacrylique comportant un ou plusieurs groupes halogénés, en particulier chlorés et/ou fluorés, et/ou comportant un groupe absorbant dans l'UVA et/ou l'UVB et pouvant apporter après polymérisation, une certaine photoprotection contre le rayonnement ultraviolet, en particulier solaire, par exemple les groupements benzylidène camphre et benzotriazole, substitués ou non, parmi lesquels le 2-(2'-hydroxy-5-methacrylyloxyéthylphényl)-2-H-benzotriazole, seul ou en mélange.

4. Composition selon la revendication 3, dans laquelle le groupe siloxane est choisi parmi
. le monomère de formule CH₂=C(CH₃)-C(O)-O-(CH₂)₃-Si-[O-Si(CH₃)₃]₃
. un macromonomère siliconé à terminaison monofonctionnelle vinylique, allylique, ester éther ou amide de l'acide acrylique ou méthacrylique, de formule
CH₂=C(R1)-C(O)-X-(CH₂)ₚ-[Si(CH₃)(R4)-O-]ₙ-Si(CH₃)₂-R3
dans laquelle R1 représente H ou CH₃, X représente O ou NH, p est un entier pouvant être nul, R3 et R4 représentent de manière indépendante CH₃ ou un groupe aliphatique, cycloaliphatique ou aromatique, et n est un entier.

5. Composition selon l'une des revendications précédentes, dans laquelle la dispersion aqueuse de polyester est choisie parmi une dispersion aqueuse de polyester anionique, cationique, non ionique ou amphotère; de polyesters à groupement ionisables latéraux, tels que sulfonique ou carboxylique; de polyesters à groupements insaturés; seul ou en mélange.

6. Composition selon l'une des revendications précédentes, se présentant sous forme d'une laque aérosol, d'un shampooing, d'une lotion de mise en plis, d'une lotion de coiffage, ou d'une mousse de coiffage.

7. Composition selon l'une des revendications précédentes, se présentant sous forme d'un produit destiné à la photoprotection des cheveux contre le rayonnement ultraviolet, en particulier contre le rayonnement solaire.

8. Utilisation d'une dispersion aqueuse de polymère constituée par des particules résultant de la polymérisation radicalaire d'au moins un monomère radicalaire à l'intérieur et/ou partiellement en surface, de particules préexistantes d'au moins un polymère choisi dans le groupe constitué par les polyesters, les polyesteramides et les alkydes, comme agent filmogène dans une composition capillaire.

9. Utilisation selon la revendication 8, dans une laque aérosol, un shampooing, une lotion de mise en plis, une lotion de coiffage ou une mousse de coiffage.

10. Utilisation selon l'une des revendications 8 à 9, dans des produits destinés à la photoprotection des cheveux contre le rayonnement ultraviolet, en particulier contre le rayonnement solaire.

## Claims

1. Hair-care composition comprising an aqueous polymer dispersion consisting of particles resulting from the free-radical polymerization of at least one radical monomer in the interior and/or partially at the surface of already existing particles of at least one polymer chosen from the group consisting of polyesters, polyesteramides and alkyds.

2. Composition according to Claim 1, in which the monomer is chosen from the group consisting of vinyl and acrylic compounds.

3. Composition according to one of the preceding claims, in which the monomer is chosen from esters of acrylic or methacrylic acids; N-substituted or N,N-substituted acrylamides or methacrylamides; vinyl esters; styrene; vinyl, acrylic or methacrylic monomers comprising one or more siloxane groups; vinyl or allyl monomers or acrylic or methacrylic acid ester, ether or amide monomers containing one or more halogenated groups, especially chlorinated and/or fluorinated groups and/or containing a group which absorbs in UVA and/or UVB and which is able to provide, after polymerization, a certain photoprotection against ultraviolet radiation, especially solar radiation, for example substituted or unsubstituted benzylidenecamphor and benzotriazole groups, including 2-(2'-hydroxy-5-methacryloyloxyethylphenyl)-2H-benzotriazole, individually or in a mixture.

4. Composition according to Claim 3, in which the siloxane group is chosen from
• the monomer of formula CH₂=C(CH₃)-C(O)-O-(CH₂)₃-Si-[O-Si(CH₃)₃]₃
• a silicon-containing macromonomer with a monofunctional vinyl, allyl, methacrylic or acrylic acid ester, ether or amide end group, of formula
CH₂=C(R1)-C(O)-X-(CH₂)ₚ-[Si(CH₃)(R4)-O-]ₙ-Si(CH₃)₂-R3
in which R1 represents H or CH₃, X represents O or NH, p is an integer which may be zero, R3 and R4 independently represent CH₃ or an aliphatic, cycloaliphatic or aromatic group, and n is an integer.

5. Composition according to one of the preceding claims, in which the aqueous polyester dispersion is chosen from an aqueous dispersion of an anionic, cationic, non-ionic or amphoteric polyester; of polyesters with ionizable side groups, such as sulpho or carboxyl groups; and of polyesters containing unsaturated groups; individually or in a mixture.

6. Composition according to one of the preceding claims, which is present in the form of an aerosol lacquer, a shampoo, a hair-setting lotion, a styling lotion or a styling mousse.

7. Composition according to one of the preceding claims, which is present in the form of a product intended for the photoprotection of hair against ultraviolet radiation, in particular against solar radiation.

8. Use of an aqueous polymer dispersion consisting of particles resulting from the free-radical polymerization of at least one radical monomer in the interior and/or partially at the surface of already existing particles of at least one polymer chosen from the group consisting of polyesters, polyesteramides and alkyds as film-forming agent in a hair-care composition.

9. Use according to Claim 8, in an aerosol lacquer, a shampoo, a hair-setting lotion, a styling lotion or a styling mousse.

10. Use according to either of Claims 8 and 9, in products intended for the photoprotection of hair against ultraviolet radiation, in particular against solar radiation.

## Patentansprüche

1. Zusammensetzung zur Haarbehandlung, die eine wäßrige Polymerdispersion enthält, die aus Partikeln besteht, die bei der radikalischen Polymerisation mindestens eines radikalischen Monomers im Inneren und/oder teilweise an der Oberfläche von bereits vorhandenen Partikeln aus mindestens einem Polymer entstehen, das unter Polyestern, Polyesteramiden und Alkydharzen ausgewählt ist.

2. Zusammensetzung nach Anspruch 1, wobei das Monomer unter Vinyl- und Acrylmonomeren ausgewählt ist.

3. Zusammensetzung nach einem der vorhergehenden Ansprüche, wobei das Monomer ausgewählt ist unter Acrylsäureestern und Methacrylsäureestern; N-substituierten und N,N-substituierten Acrylamiden und Methacrylamiden; Vinylestern; Styrol; Vinyl-, Acryl- und Methacrylmonomeren, die eine oder mehrere Siloxangruppen enthalten; Vinlymonomeren, Allylmonomeren und Estern und Amiden von Acrylsäure oder Methacrylsäure, die eine oder mehrere halogenhaltige, insbesondere chlor- und/oder fluorhaltige Gruppen, enthalten und/oder eine im UV-A- und/oder UV-B-Bereich absorbierende Gruppe enthalten, die nach der Polymerisation für einen bestimmten Lichtschutz vor UV-Strahlung, insbesondere vor dem Sonnenlicht, sorgen kann, beispielsweise die ggf. substituierte Benzylidencampher-Gruppe und die ggf. substituierte Benzotriazol-Gruppe, von denen 2-(2'-Hydroxy-5-methacrylyloxyethylphenyl)-2-H-benzotriazol genannt werden kann, die allein oder im Gemisch verwendbar sind.

4. Zusammensetzung nach Anspruch 3, wobei die Siloxangruppe ausgewählt ist unter
- dem Monomer der Formel
CH₂=C(CH₃)-C(O)-O-(CH₂)₃-Si-[O-Si(CH₃)₃]₃ und
- siliconhaltigen Makromonomeren mit einer funktionellen Vinyl- oder Allyl-Endgruppe oder einer funktionellen Endgruppe eines Esters oder Amids der Acrylsäure oder Methacrylsäure der Formel
CH₂=C(R1)-C(O)-X-(CH₂)ₚ-[Si(CH₃)(R4)-O-]ₙ-Si(CH₃)₂-R3,
worin bedeuten:
- R1 Wasserstoff oder -CH₃,
- X Sauerstoff oder -NH-,
- p eine ganze Zahl, die Null sein kann,
- R3 und R4 unabhängig voneinander -CH₃ oder eine aliphatische, cycloaliphatische oder aromatische Gruppe und
- n eine ganze Zahl.

5. Zusammensetzung nach einem der vorhergehenden Ansprüche, wobei die wäßrige Polyesterdispersion ausgewählt ist unter wäßrigen Dispersionen von anionischen, kationischen, nichtionischen oder amphoteren Polyestern, wäßrigen Dispersionen von Polyestern mit seitlich vorhandenen ionisierbaren Gruppen, wie z.B. Sulfo- oder Carboxygruppen, und wäßrigen Dispersionen von Polyestern mit ungesättigten Gruppen, die allein oder im Gemisch verwendbar sind.

6. Zusammensetzung nach einem der vorhergehenden Ansprüche, die als Aerosolspray oder -lack, Haarwaschmittel, Lotion für Wasserwellfrisuren, Frisierlotion oder Frisierschaum vorliegt.

7. Zusammensetzung nach einem der vorhergehenden Ansprüche, die in Form eines Produkts vorliegt, das für den Lichtschutz der Haare vor UV-Strahlung, insbesondere vor dem Sonnenlicht, bestimmt ist.

8. Verwendung einer wäßrigen Polymerdispersion, die aus Partikeln besteht, die bei der radikalischen Polymerisation mindestens eines radikalischen Monomers im Inneren und/oder teilweise an der Oberfläche von bereits vorhandenen Partikeln aus mindestens einem Polymer entstehen, das unter Polyestern, Polyesteramiden und Alkydharzen ausgewählt ist, als filmbildendes Mittel in einer Zusammensetzung zur Haarbehandlung.

9. Verwendung nach Anspruch 8 in Aerosolsprays oder -lacken, Haarwaschmitteln, Lotionen für Wasserwellfrisuren, Frisierlotionen und Frisierschäumen.

10. Verwendung nach einem der Ansprüche 8 und 9 in Produkten, die für den Lichtschutz der Haare vor UV-Strahlung, insbesondere vor dem Sonnenlicht, bestimmt sind.
